# EUROPÄISCHE PATENTSCHRIFT

(11) **EP 1 854 783 B1**
(45) Veröffentlichungstag und Bekanntmachung des Hinweises auf die Patenterteilung: **19.11.2014**
(21) Anmeldenummer: 07008910.7
(22) Anmeldetag: 03.05.2007
(51) Int. Cl.: C07C 263/10, C07C 265/14

(54) **Verfahren zur Herstellung von Isocyanaten**
Method for manufacturing isocyanates
Procédé destiné à la fabrication d'isocyanates

(30) Priorität: 13.05.2006 DE 102006022448
(43) Veröffentlichungstag der Anmeldung: 14.11.2007
(73) Patentinhaber: Bayer MaterialScience AG, 51368 Leverkusen (DE)
(72) Erfinder: Keggenhoff, Berthold, Dr., 47839 Krefeld (DE); Lokum, Heinrich, 50170 Kerpen (DE); Böhm, Matthias, 51373 Leverkusen (DE)

(56) Entgegenhaltungen:
- EP-A- 1 371 636
- WO-A-99/54289
- DE-A1- 19 942 299

## Beschreibung

Die Erfindung betrifft ein Verfahren zur Herstellung von Di- und Polyisocyanaten der Diphenylmethanreihe (MDI), durch Umsetzung der in einem Lösungsmittel gelösten entsprechenden Amine mit Phosgen, anschließende Abtrennung von Chlorwasserstoff und überschüssigem Phosgen, anschließende destillative Auftrennung der so erhaltenen, MDI enthaltenden Rohlösung in MDI und Lösungsmittel, Rückführung des Lösungsmittels und Herstellung von Lösungen der Amine und von Phosgen, wobei der Anteil des Lösungsmittels, der zur Herstellung der Lösung der Amine eingesetzt wird, geringe Gehalte an Phosgen und Diisocyanaten der Diphenylmethanreihe ausweist.

Die Herstellung von Isocyanaten durch Umsetzung von primären Aminen mit Phosgen ist seit längerem aus dem Stand der Technik hinlänglich bekannt, wobei in der Regel eine Lösung des Amins in einem geeigneten Lösungsmittel mit einer Lösung von Phosgen in demselben Lösungsmittel zur Reaktion gebracht wird. Verfahren zur Herstellung von organischen Isocyanaten aus primären Aminen und Phosgen sind in der Literatur beschrieben, zum Beispiel in Ullman's Encyclopedia of Industrial Chemistry, 5th ed. Vol. A 19 p. 390 ff., VCH Verlagsgesellschaft mbH, Weinheim, 1991 und G. Oertel (Ed.) Polyurethane Handbook, 2nd Edition, Hanser Verlag, München, 1993, p. 60 ff. sowie G. Wegener et. Al. Applied Catalysis A: General 221 (2001), p. 303 - 335, Elsevier Science B.V.

DE-A-19942299 beschreibt ein Verfahren zur Herstellung von Mono- und Oligoisocyanaten durch Phosgenierung der entsprechenden Amine, wobei eine katalytische Menge eines Monoisocyanats in einem inerten Lösungsmittel mit Phosgen vorgelegt wird, das Amin, normalerweise in Lösungsmittel gelöst, zugesetzt wird und das erhaltene Reaktionsgemisch mit Phosgen umgesetzt wird. Das Verfahren ist, vor allem durch Einsatz des zusätzlichen Monoisocyanates, das später wieder abgetrennt werden muss, vergleichsweise kompliziert. Eine Lehre für die erforderliche Reinheit des Lösungsmittels ist nicht zu entnehmen.

EP-A-1 073 628 beschreibt ein Verfahren zur Herstellung von Mischungen von Diphenylmethandiisocyanaten und Polyphenyl-polymethylen-polyisocyanaten (sog. polymeres MDI) durch zweistufige Umsetzung der Mischung der entsprechenden Amine mit Phosgen in Gegenwart eines Lösungsmittels unter Einhaltung ausgewählter Verhältnisse von Phosgen und Chlorwasserstoff in der zweiten Verfahrensstufe. Nach der zweistufigen Umsetzung des Amins mit Phosgen in dem ausgewählten Lösungsmittel werden das überschüssige Phosgen, der Chlorwasserstoff und das Lösungsmittel vom Reaktionsprodukt (MDI) destillativ abgetrennt. Dabei weist EP-A-1 073 628 darauf hin, dass es für eine gute Produktqualität vorteilhaft ist, dass der Restgehalt an Phosgen in der Reaktionslösung nach der Phosgenentfernung <10 ppm beträgt. Eine Lehre für die erforderliche Reinheit des Kreislauflösungsmittels ist ebenfalls nicht zu entnehmen.

Obwohl es in der Literatur des Standes der Technik zumeist nicht speziell aufgeführt ist, ist es allgemein bekannt, dass das abdestillierte Lösungsmittel zur Herstellung der Amin- und Phosgenlösung im Kreis geführt werden kann.

WO 99/542 89 beschreibt ein Verfahren zur Herstellung von Mischungen aus Diphenylmethandiisocyanaten und Polyphenylen - Polymethylen - Polyisocyanaten.

Nun wurde gefunden, dass die Reinheit des im Kreislauf geführten Lösungsmittels, das zur Herstellung der in der Phosgenierung eingesetzten Aminlösung verwendet wird, für die Nebenproduktbildung im Roh-Isocyanat von entscheidender Bedeutung ist: Selbst ein Gehalt von nur 100 ppm Phosgen oder 100 ppm Diisocyanat, bezogen auf das Gewicht des Lösungsmittels, führt zu nachweisbarer Nebenproduktbildung im Roh-Isocyanat. Während dies bei destillierten Isocyanaten, d.h. bei den als Kopfprodukt gewonnen Isocyanaten, zu einer Ausbeuteverminderung führt, wird bei den als Sumpfprodukt gewonnenen Di- und Polyisocyanaten der Diphenylmethanreihe (MDI) dadurch eine unerwünschte Beeinflussung der Qualität und des Reaktionsverhaltens bewirkt. Dies ist beispielsweise durch chlorierte Nebenkomponenten sowie erhöhten Eisengehalt nachweisbar.

Weiterhin wurde gefunden, dass das bei der Aufarbeitung und Auftrennung der MDI-Rohlösung zurückgewonnene Lösungsmittel einige hundert ppm freies Phosgen, bezogen auf das Gewicht des Lösungsmittels, enthält. Dies ist sogar selbst dann der Fall, wenn die MDI-Rohlösung vorab soweit von Phosgen befreit wird, dass kein freies Phosgen mehr nachweisbar ist. Anscheinend wird also bei der Aufarbeitung Phosgen aus Nebenkomponenten gebildet bzw. abgespalten.

Es stellt sich also die Aufgabe, ein Verfahren zur Herstellung von MDI unter Nutzung einer Lösungsmittelrückführung zur Verfügung zu stellen, bei dem die Nebenproduktbildung und damit die Ausbeuteverluste und Qualitätsbeeinträchtigung des erzeugten MDI minimiert sind.

Gegenstand der Erfindung ist ein Verfahren zur Herstellung von MDI durch Phosgenierung der entsprechenden Amine in Gegenwart eines Lösungsmittels, bei dem
a) eine Lösung von Amin in dem Lösungsmittel hergestellt wird und eine Lösung von Phosgen in dem gleichen Lösungsmittel hergestellt wird, und
b) die Lösung von Amin in dem Lösungsmittel und die Lösung von Phosgen in dem Lösungsmittel miteinander vermischt werden und das Amin mit dem Phosgen zu MDI umgesetzt werden, wobei eine MDI enthaltende Reaktionslösung erhalten wird, und
c) aus der MDI enthaltenden Reaktionslösung Chlorwasserstoff und überschüssiges Phosgen abgetrennt werden, wobei eine MDI- Rohlösung erhalten wird, und
d) die MDI- Rohlösung destillativ aufgetrennt wird, wobei in einer Destillationsstufe (4) ein MDI enthaltender Strom und ein schwach phosgenhaltiger Lösungsmittel enthaltender Strom mit einem Gehalt an Diisocyanaten der Diphenylmethanreihe von < 100 ppm und einem Restphosgengehalt von 100 - 1000 ppm, jeweils bezogen auf das Gewicht des schwach phosgenhaltigen Lösungsmittel enthaltenden Stromes, erhalten werden, und wobei
   in einer Lösungsmittelreinigung (6) der schwach phosgenhaltige Lösungsmittel enthaltende Strom aus der Destillationsstufe (4) in einer Strippkolonne destillativ gereinigt wird, wobei ein entphosgenierter Lösungsmittel enthaltender Strom mit einem Gehalt an Diisocyanaten der Diphenylmethanreihe von < 100 ppm und einem Phosgengehalt von < 100 ppm, jeweils bezogen auf das Gewicht des schwach phosgenhaltigen Lösungsmittel enthaltenden Stromes, erhalten wird, und
e) der in d) erhaltene entphosgenierte Lösungsmittel enthaltende Strom aus der Lösungsmittelreinigung (6) zumindest teilweise in Schritt a) zurückgeführt und dort zumindest für die Herstellung der Lösung von Amin in dem Lösungsmittel eingesetzt wird

Als organische Amine eignen sich 4,4'-, 2,4'- oder 2,2'-Diphenylmethandiamin oder deren Gemische, wie auch höhermolekulare isomere, oligomere oder polymere Derivate der genannten Amine.

Amine für das erfindungsgemäße Verfahren sind also die Di- und Polyamine der Diphenylmethan-Reihe (MDA, monomere, oligomere und polymere Amine). Bei der Phosgenierung erhält man die Di- und Polyisocyanate der Diphenylmethan-Reihe (MDI).

Für den Einsatz in dem erfindungsgemäßen Verfahren geeignete Lösungsmittel sind beispielsweise chlorierte aromatische Kohlenwasserstoffe, wie beispielsweise Chlorbenzol, o-Dichlorbenzol, p-Dichlorbenzol, Trichlorbenzole, die entsprechenden Chlortoluole oder Chlorxylole, Chlorethylbenzol, Monochlordiphenyl, α- bzw. β-Naphthylchlorid, Benzoesäureethylester, Phthalsäuredialkylester, Diisodiethylphthalat, Toluol und Xylole sowie Methylenchlorid, Perchlorethylen, Trichlorfluormethan und / oder Butylacetat. Gemische dieser beispielhaft genannten Lösemittel können ebenfalls eingesetzt werden. Weitere Beispiele für geeignete Lösemittel sind aus dem Stand der Technik bekannt.

Bevorzugt werden Chlorbenzol, Dichlorbenzol und Toluol als Lösungsmittel eingesetzt.

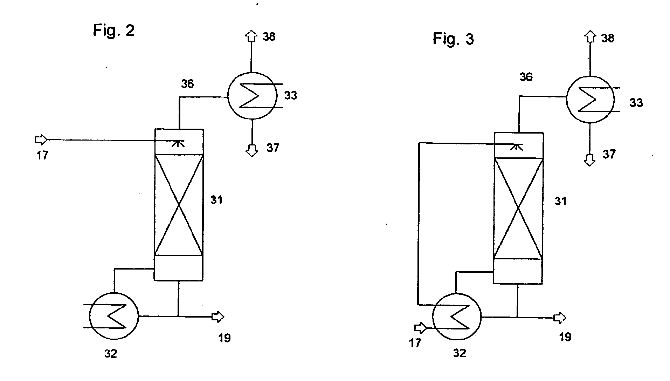

In einer bevorzugten Ausführungsform des Verfahrens wird bei der destillativen Abtrennung der Restmengen an Phosgen in der Lösungsmittelreinigung 6 die fühlbare Wärme des wiedergewonnenen Lösungsmittelstroms ganz oder teilweise als Energiequelle für diesen Abtreimungsschritt verwendet. Dies kann beispielsweise dadurch erfolgen, dass der Zulauf in die Destillationskolonne über einen Wärmeaustauscher den Kolonnensumpf beheizt. Eine geeignete Variante dieser Ausführungsform des erfindungsgemäßen Verfahrens ist in Figur 3 gezeigt. Da normalerweise das destillativ abgetrennte Lösungsmittel bei einer Temperatur von > 100 °C anfällt, das zur Erzeugung der Lösung von Amin in dem Lösungsmittel dagegen für optimale Phosgenierbedingungen < 50 °C haben sollte, kann so die Abtrennung der Restmengen an Phosgen gleichzeitig mit einer Abkühlung des Lösungsmittels verbunden werden.

Bevorzugt enthält der MDI enthaltende Strom mindestens 95 Gew.-% an MDI. bezogen auf das Gewicht des MDI enthaltenden Stroms. Bevorzugt enthält der Lösungsmittel enthaltende Strom mindestens 95 Gew.-% an Lösungsmittel, bezogen auf das Gewicht des Lösungsmittel enthaltenden Stroms.

Das erfindungsgemäße Verfahren wird nachfolgend anhand der Figuren beispielhaft näher erläutert. Es zeigen:
- Figur 1: eine schematische Darstellung des erfindungsgemäßen Verfahrens, und
- Figur 2: eine schematische Darstellung der destillativen Reinigung des Lösungsmittel enthaltenden Stroms, und
- Figur 3: eine schematische Darstellung einer alternativen, energetisch besonders günstigen destillativen Reinigung des Lösungsmittel enthaltenden Stroms.

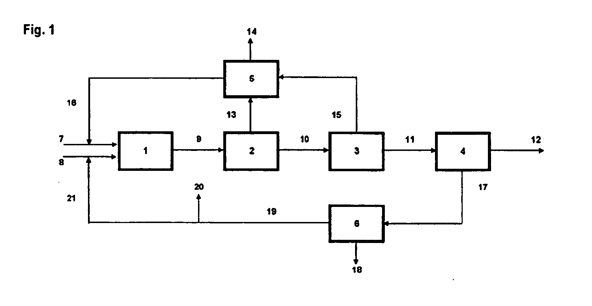

In Fig. 1 ist das erfindungsgemäße Verfahren zur Herstellung von Isocyanaten beispielhaft schematisch dargestellt.

Dabei bedeuten Stufe 1 die Vorphosgenierstufe (Mischer 1) und Stufe 2 die Heißphosgenierstufe (Phosgenierreaktor 2). Die Stufen 1 und 2 entsprechen Schritt b) des erfindungsgemäßen Verfahrens. In der Stufe 3 (Entphosgenierstufe 3) wird aus der MDI enthaltenden Reaktionslösung Chlorwasserstoff und überschüssiges Phosgen abgetrennt (Schritt c)). Dabei wird technisch bevorzugt ein großer Teil des gebildeten Chlorwasserstoffs zusammen mit dem überschüssigen Phosgen bereits direkt beim Austritt aus dem Phosgenierreaktor 2 abgetrennt, ein weiterer Teil in einer Entphosgenierkolonne. In der Stufe 4 (Destillationsstufe 4) wird die aus Entphosgenierstufe 3 erhaltene MDI-Rohlösung weiter aufgearbeitet und MDI und Lösungsmittel destillativ getrennt (Schritt d)). In der Stufe 6 (Lösungsmittelreinigung 6) erfolgt dann die destillative Reinigung des in Stufe 4 erhaltenen Lösungsmittel enthaltenden Stroms zur Abtrennung von Restphosgenmengen aus dem Kreislauflösungsmittel. In der Stufe 5 (Brüdenkolonne 5) erfolgt die Aufarbeitung der in den Stufen 2 und 3 erhaltenen Brüden (i. W. Rückgewinnung von Phosgen und anteilig von Lösungsmittel).

Aus frischem Phosgen (Strom 7) und zurückgeführtem Überschussphosgen sowie phosgenhaltigem Lösungsmittel (Strom 16) wird die Lösung von Phosgen in dem Lösungsmittel (Phosgenlösung) hergestellt. Parallel dazu wird aus dem Amin (Strom 8) und dem zurückgeführten, von MDI und Phosgen weitgehend befreiten Lösungsmittelstrom (Strom 21) die Lösung von Amin in dem Lösungsmittel (Aminlösung) hergestellt. Natürlich kann eine der Lösungen zumindest anteilig auch mit frischem Lösungsmittel hergestellt werden. Die Phosgenlösung und die Aminlösung werden in dem Mischer 1 unter intensivem Vermischen zur Reaktion gebracht und die so erhaltene Mischung (Strom 9) in dem Phosgenierreaktor 2 durch Erhitzen unter Abspaltung von Chlorwasserstoff in die MDI enthaltende Reaktionslösung (Strom 10) umgesetzt. Diese wird in der Entphosgenierstufe 3 destillativ von Restmengen an Phosgen befreit und als praktisch phosgenfreie Isocyanat-Rohlösung (Strom 11) in die Destillationsstufe 4 geführt. Die in den Stufen 2 und 3, d.h. im Phosgenierreaktor 2 und der Entphosgenierstufe 3, erhaltenen Brüden (Ströme 13 und 15), die im Wesentlichen aus Chlorwasserstoff, Überschussphosgen und Lösungsmittelanteilen bestehen, werden in der Brüdenkolonne 5 in Chlorwasserstoff (Strom 14) und Überschussphosgen in Lösungsmittel (Strom 16) aufgetrennt. Der Chlorwasserstoff (Strom 14) wird ausgeschleust und bevorzugt einer weiteren Verwertung zugeführt.

In der Destillationsstufe 4 wird die MDI-Rohlösung (Strom 11) in MDI (MDI enthaltender Strom 12) und das rückgewonnene Lösungsmittel (Lösungsmittel enthaltender Strom 17) destillativ getrennt. Da MDI normalerweise einen höheren Siedepunkt als das Lösungsmittel hat, kann durch geeignete Gestaltung der Aufarbeitung in der Destillationsstufe 4 gewährleistet werden, dass das Lösungsmittel (Lösungsmittel enthaltender Strom 17) den geforderten geringen Diisocyanatgehalt von < 100 ppm, bevorzugt < 50 ppm, besonders bevorzugt < 20 ppm, bezogen auf das Gewicht des Lösungsmittel enthaltenden Stroms, besitzt.

Da aber in der Aufarbeitung in der Destillationsstufe 4 Phosgen aus Nebenkomponenten der Phosgenierung rückgespalten wird, hat der Lösungsmittel enthaltende Strom (Strom 17) stets einen Restgehalt an Phosgen. Dieser wird nun in der Lösungsmittelreinigung 6 als phosgenangereicherter Lösungsmittelstrom (Strom 18) abgetrennt und kann in den Prozess zurückgeführt werden und beispielsweise dem Strom 16 zugeschlagen werden (nicht dargestellt in Figur 1). Der gereinigte Lösungsmittel enthaltende Strom 19 mit einem Phosgengehalt von < 100 ppm, bevorzugt < 50 ppm, besonders bevorzugt < 20 ppm, und einem Diisocyanatgehalt von < 100 ppm, bevorzugt < 50 ppm, besonders bevorzugt < 20 ppm, jeweils bezogen auf das Gewicht des Lösungsmittel enthaltenden Stroms, kann teilweise als Strom 20 ausgeschleust und an anderer Stelle im Prozess verwendet werden, wird aber zumindest teilweise, bevorzugt überwiegend als Strom 21 zur Herstellung der Aminlösung eingesetzt.

Die Umsetzung der Aminlösung mit der Phösgenlösung in Schritt b) (Stufen 1 und 2) erfolgt üblicherweise bei Temperaturen von 20 bis 240°C und absoluten Drücken von 1 bis 50 bar. Sie kann einstufig oder mehrstufig durchgeführt werden, wobei Phosgen üblicherweise im stöchiometrischen Überschuss eingesetzt wird. Dabei werden in Stufe 1 die Aminlösung und die Phosgenlösung bevorzugt über statische Mischelemente oder spezielle dynamische Mischelemente vereinigt und anschließend in Stufe 2 beispielsweise von unten nach oben durch einen oder mehrere Reaktionstürme geführt, in denen das Gemisch zu MDI reagiert. Neben Reaktionstürmen, die mit geeigneten Mischelementen versehen sind, können auch Reaktionsbehälter mit Rührvorrichtung eingesetzt werden. Geeignete statische und dynamische Mischelemente sowie Reaktionsvorrichtungen sind aus dem Stand der Technik bekannt.

Die Abtrennung von restlichem Phosgen und Chlorwasserstoff in Schritt c) aus der erhaltenen MDI enthaltenden Reaktionslösung erfolgt vorteilhaft in der Entphosgenierstufe 3, wobei die MDI enthaltenden Reaktionslösung auf den Abtriebsteil einer Destillationskolonne gegeben wird. Bevorzugt wird dieser Destillationsschritt so durchgeführt, dass die entphosgenierte MDI-Rohlösung als Sumpfprodukt mit einem Restgehalt an Phosgen von < 100 ppm, bevorzugt < 10 ppm, bezogen auf das Gewicht der Isocyanat-Rohlösung, erhalten wird.

Die destillative Auftrennung der MDI-Rohlösung in Schritt d) erfolgt in einer dem Siedepunkt des Lösungsmittels angepassten Weise in einer ein- oder bevorzugt mehrstufigen Destillationssequenz in der Destillationsstufe 4. Solche Destillationssequenzen sind aus dem Stand der Technik bekannt.

In dem bevorzugten Fall der Herstellung von MDI unter Verwendung von Monochlorbenzol als Lösungsmittel kann diese destillative Auftrennung in Schritt d) vorteilhaft so erfolgen, dass die Isocyanat-Rohlösung in zwei Schritten in ein Sumpfprodukt enthaltend mindestens 95 Gew.-%, besonders bevorzugt mindestens 97 Gew.-% an MDI, bezogen auf das Gewicht des MDI enthaltenden Stroms, aufgearbeitet wird, das anschließend bevorzugt in weiteren Schritten von Leichtsiedem befreit wird. Im ersten Schritt werden dabei bevorzugt durch eine Flashdestillation bei absoluten Drücken von 600 - 1200 mbar und Sumpftemperaturen von 110 - 170 °C 60 - 90 % des in der MDI-Rohlösung enthaltenen Lösungsmittels abgetrennt, wobei die Brüden in einer Destillationskolonne mit 5 - 20 Trennstufen und 10 - 30 % Rücklauf aufgearbeitet werden, so dass ein Lösungsmittel enthaltender Strom mit einem Diisocyanatgehalt von < 100 ppm, bevorzugt < 50 ppm, besonders bevorzugt < 20 ppm, bezogen auf das Gewicht des Lösungsmittel enthaltenden Stroms, erzielt wird. Im zweiten Schritt wird das restliche Lösungsmittel bis auf einen Restgehalt von 1 - 3 Gew. % im Sumpfprodukt bei absoluten Drücken von 60 - 140 mbar und Sumpftemperaturen von 130 - 190 °C abgetrennt. Die Brüden können ebenfalls in einer Destillationskolonne mit 5 - 20 Trennstufen und 10 - 40 % Rücklauf aufgearbeitet werden, so dass ein Lösungsmittel enthaltender Strom mit einem Diisocyanatgehalt von < 100 ppm, bevorzugt < 50 ppm, besonders bevorzugt < 20 ppm, bezogen auf das Gewicht des Lösungsmittel enthaltenden Stroms, erzielt wird oder nach Kondensation wieder als Zulauf in den ersten Destillationsschritt rückgeführt werden. In gleicher Weise können die in den folgenden Schritten abgetrennten Destillatströme wieder als Zulauf in den ersten Destillationsschritt rückgeführt werden.

Auf diese Weise kann der gesamte Lösungsmittel enthaltende Strom mit der geforderten Spezifikation bzgl. Diisocyanat (< 100 ppm an Diisocyanaten, bezogen auf das Gewicht des Lösungsmittel enthaltenden Stroms) in vorteilhafter Weise abgetrennt werden. Dieser Lösungsmittel enthaltende Strom kann aber als Verunreinigung Monoisocyanate (z. B. Phenylisocyanat) mit einem Gehalt von 100 - 1000 ppm sowie eine Restphosgenmenge von 100 - 1000 ppm enthalten.

Erfindungsgemäß wird diese Destillation nur nach dem zu erzielenden Diisocyanatgehalt von < 100 ppm, bevorzugt < 50 ppm,

besonders bevorzugt < 20 ppm ausgelegt, und der Restphosgengehalt, der dann noch 100 - 1000 ppm beträgt, wird in der separaten Stufe 6 abgetrennt.

Eine mögliche Ausführung der destillativen Lösungsmittelreinigung in Stufe 6 ist in Fig. 2 dargestellt. Die Lösungsmittelreinigung umfasst eine Strippkolonne 31, einen Sumpfverdampfer 32 und einen Kopfkondensator 33. Der schwach phosgenhaltige Lösungsmittel enthaltende Strom 17 aus der Aufarbeitung in Stufe 4 (nicht dargestellt in Figur 2) wird auf die Strippkolonne 31 aufgegeben, die bevorzugt 4 - 20 Trennstufen besitzt. Der Sumpfverdampfer 32 erzeugt durch Beheizung z.B. mit Heizdampf ausreichende Mengen an Brüden, so dass der entphosgenierte Lösungsmittel enthaltende Strom 19 nur noch einen Phosgengehalt von < 100 ppm, bevorzugt < 50 ppm, besonders bevorzugt < 20 ppm, und einen Diisocyanatgehalt von < 100 ppm, bevorzugt < 50 ppm, besonders bevorzugt < 20 ppm, jeweils bezogen auf das Gewicht des Lösungsmittel enthaltenden Stroms, besitzt und so zur Herstellung der Aminlösung verwendet werden kann. Der erzeugte Brüdenstrom 36 enthält das abgetrennte Phosgen in bevorzugt 1 - 6 Gew.-%, bezogen auf das Gewicht des Brüdenstroms, und wird bevorzugt am Kondensator 33 kondensiert; während das Kondensat 37 in den Isocyanatprozess, beispielsweise zur Bereitung der Phosgenlösung zugeführt wird, werden die Restgase 38 bevorzugt der Abgasaufarbeitung zugeführt. Das Kondensat 37 kann aber auch ganz oder teilweise als Rücklauf auf die Strippkolonne 31 rückgeführt werden, wodurch sich das Phosgen im Brüdenstrom 36 weiter aufkonzentriert. Wird die Stufe 6 bei einem Druck unterhalb der Siedetemperatur des Lösungsmittels im Lösungsmittel enthaltenden Strom 17 betrieben, tritt im Eintritt in die Strippkolonne 31 durch Ausflashen bereits eine teilweise Abtrennung von Phosgen ein. So wird die dem Verdampfer 32 zuzuführende Energiemenge reduziert.

Fig. 3 zeigt schließlich eine besonders bevorzugte, weil energetisch besonders günstige Ausführungsform der destillativen Lösungsmittelreinigung in Stufe 6:

Der schwach phosgenhaltige Lösungsmittel enthaltende Strom 17 aus der Aufarbeitung in Stufe 4 (nicht dargestellt in Figur 3) wird zunächst als Heizmittel durch den Sumpfverdampfer 32 und dann auf die Strippkolonne 31 aufgegeben, die 4 - 20 Trennstufen besitzt. Der Sumpfverdampfer 32 erzeugt durch die Beheizung mit dem Lösungsmittel enthaltenden Strom ausreichende Mengen an Brüden, so dass der das entphosgenierte Lösungsmittel enthaltende Strom 19 nur noch einen Phosgengehalt von < 100 ppm, bevorzugt < 50 ppm, besonders bevorzugt < 20 ppm, und einen Diisocyanatgehalt von < 100 ppm, bevorzugt < 50 ppm, besonders bevorzugt < 20 ppm, jeweils bezogen auf das Gewicht des Lösungsmittel enthaltenden Stroms, besitzt und so zur Herstellung der Aminlösung verwendet werden kann. Dabei wird der Lösungsmittelstrom um 2 - 10 °C abgekühlt. Der erzeugte Brüdenstrom 36 enthält das abgetrennte Phosgen in bevorzugt 1 - 6 Gew.-%, bezogen auf das Gewicht des Brüdenstroms, und wird bevorzugt am Kondensator 33 kondensiert; während das Kondensat 37 in den MDI-Prozess, beispielsweise zur Bereitung der Phosgenlösung zugeführt wird, werden die Restgase 38 bevorzugt über ein Vakuumsystem der Abgasaufarbeitung zugeführt. Das Kondensat 37 kann aber auch ganz oder teilweise als Rücklauf auf die Strippkolonne 31 rückgeführt werden, wodurch sich das Phosgen im Brüdenstrom 36 weiter aufkonzentriert. Durch Regelung des Drucks im System wird die Menge an erzeugten Brüden und dadurch die Qualität bzw. Reinheit des Lösungsmittel enthaltenden Stroms geregelt. Insgesamt wird in dieser Ausführungsform die Abtrennung der Restphosgenmengen ohne Zufuhr von Fremdenergie bewirkt, wobei sogar gleichzeitig eine normalerweise erwünschte Abkühlung des Lösungsmittel enthaltenden Stroms, der zur Herstellung der Aminlösung verwendet wird, bewirkt wird.

### Beispiele

### a) Herstellung eines Gemisches von Di- und Polyaminen:

### Beispiel 1

In einem Rührgefäß werden 2600 g Anilin bei 25°C mit 1000 g Formalin (30 Gew. %ige wässrige Lösung von Formaldehyd, bezogen auf das Gewicht der Lösung) unter Rühren intensiv vermischt, wobei sich die Mischung auf 60°C erwärmt. Man stellt den Rührer ab und trennt die sich oben abscheidende Wasserphase ab. Anschließend mischt man unter erneutem Rühren und Kühlen 68 g 30 Gew. %ige wässrige Salzsäure zu, wobei eine Temperatur von 45°C gehalten wird. Nach 15 min weiterem Rühren bei dieser Temperatur wird die Kühlung durch Heizung ersetzt und die Mischung im Lauf von 120 min unter 5 bar Druck gleichmäßig auf 140°C aufgeheizt und danach 15 min bei dieser Temperatur gehalten.

Anschließend wird die Mischung auf 100°C abgekühlt, auf Normaldruck entspannt und durch Zugabe von 54 g 50 Gew. %ige wässriger Natronlauge unter Rühren neutralisiert. Nach Abstellen des Rührers lässt man die Phasen absitzen und saugt die untere Wasserphase ab. Anschließend destilliert man zunächst unter Normaldruck überschüssiges Anilin mit verbliebenem Restwasser ab und entfernt die Anilinreste durch Andestillieren des erhaltenen Polyamingemischs bei 100 mbar und 250°C.

Man erhält 1900 g eines Gemisches von Di- und Polyaminen der folgenden Zusammensetzung:
4,4'-MDA: 60,1 Gew. %,
2,4'-MDA: 6,0 Gew. %,
2,2'-MDA: 0,2 Gew. %,
höhermolekulare Polyamine: 33,7 Gew. %, jeweils bezogen auf das Gewicht des Gemisches.

### b) Herstellung eines Gemisches von Di- und Polyisocyanaten:

### Beispiel 2 Verwendung von unreinem Lösungsmittel (nicht erfindungsgemäß)

Man löst in einem Rührreaktor die 1900 g des in Beispiel 1 erhaltenen Gemisches von Di- und Polyaminen in 5700 g Chlorbenzol mit einem Gehalt von 200 ppm Phosgen und 200 ppm MDI, jeweils bezogen auf das Gewicht des Lösungsmittels Chlorbenzol, auf. In einem zweiten Gefäß aus Edelstahl (DIN 1.4571) stellt man durch Lösen von 3800 g Phosgen in 7600 g Chlorbenzol unter Kühlen auf 0°C eine 33 Gew.%ige (bezogen auf das Gewicht der Lösung) Phosgenlösung her und vermischt in diesem unter intensivem Rühren die Amin- und Phosgenlösungen. Die entstandene Feststoffsuspension wird nun langsam aufgeheizt, wobei Chlorwasserstoffgas entsteht, das in geeigneter Weise abgeleitet wird. Dabei entsteht eine homogene Lösung des Polyisocyanats. Durch Destillation wird nun das Lösungsmittel abgetrennt, wodurch man 2370 g eines Gemisches von Di- und Polyisocyanaten der folgenden Zusammensetzung erhält:
4,4'-MDI: 59,2 Gew. %,
2,4'-MDI: 5,4 Gew. %,
2,2'-MDI: 0,2 Gew. %,
höhermolekulare Polyisocyanate: 35,2 Gew. %, jeweils bezogen auf das Gewicht des Gemisches.
Acidität (ASTM D 1638-74): 180 ppm
Eisengehalt: 10 ppm
Extinktion einer 2%igen Lösung in Chlorbenzol (Wellenlänge 430 nm, Schichtdicke 10 mm): 0,27

### Beispiel 3 Verwendung von reinem Lösungsmittel (erfindungsgemäß)

Man löst in einem Rührreaktor die 1900 g des in Beispiel 1 erhaltenen Gemisches von Di- und Polyaminen in 5700 g Chlorbenzol mit einem Gehalt von 20 ppm Phosgen und 20 ppm MDI, jeweils bezogen auf das Gewicht des Lösungsmittels Chlorbenzol, auf. In einem zweiten Gefäß aus Edelstahl (DIN 1.4571) stellt man durch Lösen von 3800 g Phosgen in 7600 g Chlorbenzol unter Kühlen auf 0°C eine 33 Gew.%ige (bezogen auf das Gewicht der Lösung) Phosgenlösung her und vermischt unter intensivem Rühren die Amin- und Phosgenlösungen. Die entstandene Feststoffsuspension wird nun langsam aufgeheizt, wobei Chlorwasserstoffgas entsteht, das in geeigneter Weise abgeleitet wird. Dabei entsteht eine homogene Lösung des Polyisocyanats. Durch Destillation wird nun das Lösungsmittel abgetrennt, wodurch man 2370 g eines Gemisches von Di- und Polyisocyanaten der folgenden Zusammensetzung erhält:
4,4'-MDI: 59,3 Gew. %,
2,4'-MDI: 5,5 Gew. %,
2,2'-MDI: 0,2 Gew. %,
höhermolekulare Polyisocyanate: 35 Gew. %, bezogen auf das Gewicht des Gemisches.
Acidität (ASTM D 1638-74): 62 ppm
Eisengehalt: 4 ppm
Extinktion einer 2%igen Lösung in Chlorbenzol (Wellenlänge 430 nm, Schichtdicke 10 mm): 0,13

Es zeigt sich somit beim Vergleich der Ergebnisse der Beispiele 2 und 3, dass durch den Einsatz von gereinigtem Lösungsmittel für die Herstellung der Aminlösung gemäß dem erfindungsgemäßen Verfahren ein Isocyanat mit verbesserter Qualität erhalten wird, was sich in einer geringen Acidität, einem geringen Eisengehalt und einer hellen Farbe (geringe Extinktion) äußert.

## Patentansprüche

1. Verfahren zur Herstellung von Di- und Polyisocyanaten der Diphenylmethanreihe (MDI) durch Phosgenierung der entsprechenden Amine in Gegenwart eines Lösungsmittels, bei dem
a) eine Lösung von Amin in dem Lösungsmittel hergestellt wird und eine Lösung von Phosgen in dem gleichen Lösungsmittel hergestellt wird, und
b) die Lösung von Amin in dem Lösungsmittel und die Lösung von Phosgen in dem Lösungsmittel miteinander vermischt werden und das Amin mit dem Phosgen zu MDI umgesetzt wird, wobei eine MDI enthaltende Reaktionslösung erhalten wird, und
c) aus der MDI enthaltenden Reaktionslösung Chlorwasserstoff und überschüssiges Phosgen abgetrennt werden, wobei eine MDI- Rohlösung erhalten wird, und
d) die MDI- Rohlösung destillativ aufgetrennt wird, wobei
in einer Destillationsstufe (4) ein MDI enthaltender Strom und ein schwach phosgenhaltiger Lösungsmittel enthaltender Strom mit einem Gehalt an Diisocyanaten der Diphenylmethanreihe von < 100 ppm und einem Restphosgengehalt von 100 - 1000 ppm, jeweils bezogen auf das Gewicht des schwach phosgenhaltigen Lösungsmittel enthaltenden Stromes, erhalten werden, und wobei
in einer Lösungsmittelreinigung (6) der schwach phosgenhaltige Lösungsmittel enthaltende Strom aus der Destillationsstufe (4) in einer Strippkolonne destillativ gereinigt wird, wobei ein entphosgenierter Lösungsmittel enthaltender Strom mit einem Gehalt an Diisocyanaten der Diphenylmethanreihe von < 100 ppm und einem Phosgengehalt von < 100 ppm, jeweils bezogen auf das Gewicht des schwach phosgenhaltigen Lösungsmittel enthaltenden Stromes, erhalten wird, und
e) der in d) erhaltene entphosgenierte Lösungsmittel enthaltende Strom aus der Lösungsmittelreinigung (6) zumindest teilweise in Schritt a) zurückgeführt und dort zumindest für die Herstellung der Lösung von Amin in dem Lösungsmittel eingesetzt wird.

2. Verfahren nach Anspruch 1, bei dem der in Schritt e) zurückgeführte entphosgenierte Lösungsmittel enthaltende Strom auch für die Herstellung der Lösung von Phosgen in dem Lösungsmittel eingesetzt wird.

3. Verfahren nach einem der Ansprüche 1 bis 2, bei dem als Lösungsmittel Chlorbenzol, Dichlorbenzol und / oder Toluol eingesetzt werden.

4. Verfahren nach einem der Ansprüche 1 bis 3, bei dem der in Schritt e) zurückgeführte entphosgenierte Lösungsmittel enthaltende Strom als Sumpfprodukt der Strippkolonne erhalten wird und der Eingangsstrom in die Strippkolonne durch Wärmeaustausch mit dem Sumpf der Strippkolonne abgekühlt wird.

## Claims

1. Process for preparing di- and polyisocyanates of the diphenylmethane series (MDI) by phosgenation of the corresponding amines in the presence of a solvent, wherein
a) a solution of amine in the solvent is prepared and a solution of phosgene in the same solvent is prepared, and
b) the solution of amine in the solvent and the solution of phosgene in the solvent are mixed with one another and the amine is reacted with the phosgene to MDI, giving a reaction solution comprising MDI, and
c) hydrogen chloride and excess phosgene are removed from the reaction solution comprising MDI, giving a crude MDI solution, and
d) the crude MDI solution is fractionally distilled,
giving, in a distillation stage (4), a stream comprising MDI and a stream comprising solvent of low phosgene content and having a diphenylmethane-series diisocyanate content of < 100 ppm and a residual phosgene content of 100-1000 ppm, based in each case on the weight of the stream comprising solvent of low phosgene content, and
in a solvent cleaning operation (6), the stream comprising solvent of low phosgene content from the distillation stage (4) being purified by distillation in a stripping column, giving a dephosgenated solvent-comprising stream having a diphenylmethane-series diisocyanate content of < 100 ppm and a phosgene content of < 100 ppm, based in each case on the weight of the stream comprising solvent of low phosgene content, and
e) the dephosgenated solvent-comprising stream obtained in d) from the solvent cleaning operation (6), being at least partly recycled to step a), where it is used at least for preparing the solution of amine in the solvent.

2. Process according to Claim 1, wherein the dephosgenated solvent-comprising stream recycled in step e) is also used for preparing the solution of phosgene in the solvent.

3. Process according to one of Claims 1 to 2, wherein chlorobenzene, dichlorobenzene and/or toluene are used as solvents.

4. Process according to any of Claims 1 to 3, wherein the dephosgenated solvent-comprising stream recycled in step e) is obtained as the liquid-phase product of the stripping column, and the entering stream into the stripping column is cooled by heat exchange with the liquid phase of the stripping column.

## Revendications

1. Procédé pour la préparation de diisocyanate et de polyisocyanates de la série du diphénylméthane (MDI) par phosgénation des amines correspondantes en présence d'un solvant, dans lequel
a) on prépare une solution d'amine dans le solvant et on prépare une solution de phosgène dans le même solvant et
b) on mélange, l'une avec l'autre, la solution d'amine dans le solvant et la solution de phosgène dans le solvant et l'amine est transformée avec le phosgène en MDI, avec obtention d'une solution réactionnelle contenant du MDI et
c) on sépare le chlorure d'hydrogène et le phosgène en excès de la solution réactionnelle contenant du MDI, avec obtention d'une solution brute de MDI et
d) la solution brute de MDI est séparée par distillation,
avec obtention, dans une étape de distillation (4), d'un flux contenant du MDI et d'un flux contenant le solvant à faible teneur en phosgène présentant une teneur en diisocyanates de la série du diphénylméthane < 100 ppm et une teneur résiduelle en phosgène de 100-1000 ppm, à chaque fois par rapport au poids du flux contenant le solvant à faible teneur en phosgène,
en purifiant, dans une purification du solvant (6), le flux contenant le solvant à faible teneur en phosgène provenant de l'étape de distillation (4), par distillation dans une colonne de rectification, avec obtention d'un flux contenant le solvant dont on a éliminé le phosgène présentant une teneur en diisocyanate de la série du diphénylméthane < 100 ppm et une teneur en phosgène de < 100 ppm, à chaque fois par rapport au flux contenant le solvant à faible teneur en phosgène, et
e) le flux contenant le solvant dont on a enlevé le phosgène obtenu dans l'étape d) de la purification du solvant (6) étant recyclé au moins partiellement dans l'étape a) et étant utilisé dans celle-ci au moins pour la préparation de la solution d'amine dans le solvant.

2. Procédé selon la revendication 1, dans lequel le flux recyclé contenant le solvant dont on a enlevé le phosgène de l'étape e) est également utilisé pour la préparation de la solution de phosgène dans le solvant.

3. Procédé selon l'une quelconque des revendications 1 à 2, dans lequel on utilise comme solvant du chlorobenzène, du dichlorobenzène et/ou du toluène.

4. Procédé selon l'une quelconque des revendications 1 à 3, dans lequel le flux recyclé contenant le solvant dont on a enlevé le phosgène de l'étape e) est obtenu sous forme de produit du fond de la colonne de rectification et le flux entrant dans la colonne de rectification est refroidi par échange thermique avec le fond de la colonne de rectification.
